**Europäisches Patentamt**

**European Patent Office**

**Office européen des brevets**

(19)

(11) Veröffentlichungsnummer : **0 353 501 B1**

(12) **EUROPÄISCHE PATENTSCHRIFT**

(45) Veröffentlichungstag der Patentschrift :
**21.09.94 Patentblatt 94/38**

(21) Anmeldenummer : **89112704.5**

(22) Anmeldetag : **12.07.89**

(51) Int. Cl.$^5$ : **G01N 33/52**

(54) Testträger zur analytischen Bestimmung eines Bestandteils einer flüssigen Probe.

(30) Priorität : **30.07.88 DE 3826056**

(43) Veröffentlichungstag der Anmeldung :
**07.02.90 Patentblatt 90/06**

(45) Bekanntmachung des Hinweises auf die
Patenterteilung :
**21.09.94 Patentblatt 94/38**

(84) Benannte Vertragsstaaten :
**AT BE CH DE ES FR GB GR IT LI LU NL SE**

(56) Entgegenhaltungen :
**EP-A- 0 267 519**
**EP-A- 0 353 500**

(73) Patentinhaber : **BOEHRINGER MANNHEIM GMBH**
**D-68298 Mannheim (DE)**

(72) Erfinder : **Schlipfenbacher, Reiner, Dr.**
**Beethovenstrasse 9**
**D-6840 Lampertheim (DE)**
Erfinder : **Steinbiss, Joachim, Dr.**
**Alexanderstrasse 21a**
**D-6143 Lorsch (DE)**
Erfinder : **Trasch, Heinz Friedrich, Dr.**
**Wissmannstrasse 14**
**D-6700 Ludwigshafen (DE)**

(74) Vertreter : **Pfeifer, Hans-Peter, Dr.rer.nat.**
**Patentanwalt**
**Nowackanlage 15**
**D-76137 Karlsruhe (DE)**

EP 0 353 501 B1

## Beschreibung

Die Erfindung betrifft einen Testträger zur analytischen Bestimmung eines Bestandteils einer flüssigen Probe, insbesondere für die Diagnose von Krankheiten mittels einer auf dem Testträger ablaufenden Reaktionsfolge. Der Testträger weist mehrere Testschichten auf. Hierzu gehören eine Basisschicht, eine Flüssigkeitstransportstrecke, die sich von einem Probenaufgabebereich in einen Auswertebereich erstreckt und eine Signalbildungsschicht, in der aufgrund einer Signalbildungsreaktion mit Hilfe eines Signalbildungs-Reagenzsystems eine für den zu bestimmenden Bestandteil charakteristische optisch nachweisbare Veränderung stattfindet.

Für qualitative oder quantitative analytische Bestimmungen im Rahmen der Diagnose von Krankheiten werden in jüngerer Zeit zunehmend sogenannte trägergebundene Tests verwendet. Bei diesen sind Reagenzien in entsprechende Schichten eines festen Testträgers eingebettet, der mit der Probe in Kontakt gebracht wird. Die Probe ist meist eine Körperflüssigkeit wie Blut oder Urin. Es kann sich aber auch um eine durch vorausgehende Testschritte, beispielsweise durch Kontaktierung eines Eluationsmittels mit einer Stuhlprobe, gewonnene Flüssigkeit handeln.

Die Reaktion der flüssigen probe mit den Reagenzien führt zu einem nachweisbaren Signal, wobei sich die Erfindung auf Fälle bezieht, bei denen ein optisch nachweisbares Signal erzeugt wird. Meist handelt es sich dabei um eine Farbänderung, die in der Signalbildungsschicht stattfindet. Andere optisch nachweisbare Signalbildungsreaktionen führen beispielsweise zu einem Fluoreszenzsignal. Die optisch nachweisbare Veränderung kann visuell oder mit Hilfe eines entsprechenden Gerätes, meistens reflexionsphotometrisch, ausgewertet werden.

Es werden verschiedene Signalbildungsreaktionen verwendet, die aus mehreren Reaktionsstufen bestehen können, an denen mehrere verschiedene Reagenzien beteiligt sind. Diese werden insgesamt als Signalbildungs-Reagenzsystem bezeichnet. Mindestens ein Signalbildungs-Reagenz dieses Systems befindet sich in der Signalbildungsschicht und nimmt an einer Reaktionsstufe teil, die zu der optisch nachweisbaren Veränderung führt.

Testträger sind in vielen verschiedenen Gestaltungen bekannt. Die Erfindung richtet sich auf solche Testträger, die eine in der Regel langgestreckte Basisschicht aufweisen, auf der man einen Probenaufgabebereich und einen Auswertebereich unterscheiden kann. Bei derartigen Testträgern wird die Probe im Probenaufgabebereich aufgegeben und dann längs einer Flüssigkeitstransportstrecke, die parallel zu der Basisschicht verläuft, in den Auswertebereich transportiert. Derartige "Testträger mit Längstransport" haben gegenüber den klassischen Testträgern mit ausschließlich übereinander angeordneten Testschichten erhebliche Vorteile und sind vor allem für immunologische Analyseverfahren gut geeignet. Der Flüssigkeitstransport basiert auf Kapillarwirkung, wobei die Flüssigkeitstransportstrecke sowohl durch eine oder mehrere Testschichten aus saugfähigem Material, wie beispielsweise Papier oder Vlies, als auch durch einen Spalt, der sich durch Kapillarwirkung vollsaugt, gebildet werden kann. Testträger mit Längstransport sind beispielsweise in der DE-A 34 45 816, der DE-A 36 38 654 und der DE-A 36 43 516 beschrieben.

In der DE-A 34 45 816 ist die Signalbildungsschicht, die dort als Detektionszone bezeichnet wird, neben anderen Schichten, die die Flüssigkeitstransportstrecke bilden, angeordnet. Die Probenflüssigkeit durchströmt die Signalbildungsschicht dabei in deren Längsrichtung. Dadurch ergeben sich Chromatographieeffekte, die in vielen Anwendungsfällen zu einer ungleichmäßigen Farbbildung führen.

Bei dem in der DE-A 36 38 654 beschriebenen Testträger ist die Signalbildungsschicht an einer im Nachweisbereich vorgesehenen Klappe angebracht, die im Ausgangszustand des Testträgers nicht in Fluidkontakt zu der Flüssigkeitstransportstrecke steht, sondern erst durch eine äußere mechanische Manipulation (manuell oder mit Hilfe eines Gerätes) nach unten gegen eine Flüssigkeitstransportschicht gedrückt wird, die Teil der Flüssigkeitstransportstrecke ist, und in diesem Benutzungsstadium mit der Probenflüssigkeit getränkt wird. Diese Maßnahmen ermöglichen eine definierte Farbbildung zu einem bestimmten Zeitpunkt, jedoch erfordern sie die erwähnte mechanische Manipulation.

Bei dem in der DE-A 36 43 516 beschriebenen Testträger ist eine Flüssigkeitstransportstrecke vorgesehen, die von einer Probenaufgabezone bis zu einer Saugzone verläuft. An dieser, vorzugsweise durch einen Spalt gebildeten Flüssigkeitstransportstrecke ist eine Signalbildungsschicht so angeordnet, daß sie mit einer die Flüssigkeitstransportstrecke entlang strömenden Flüssigkeit in Kontakt steht. Eine definierte Farbbildung wird dabei dadurch angestrebt, daß der Strömungsvorgang in der Flüssigkeitstransportstrecke und die Saugeigenschaften der Signalbildungsschicht in bestimmter Weise aufeinander abgestimmt sind. Dadurch wird eine gut reproduzierbare Dosierung der von der Signalbildungsschicht aufgenommenen Flüssigkeit erreicht, jedoch erfordert die dort beschriebene Gestaltung eine aufwendige Herstellung.

Der Erfindung liegt die Aufgabe zugrunde, bei einem Testträger mit Längstransport definierte Bedingungen für die Farbbildungsreaktion und eine gleichmäßige Farbbildung mit geringem Aufwand zu erreichen.

2

Hierzu wird erfindungsgemäß vorgeschlagen, daß in dem Auswertebereich eine Flüssigkeitstransportschicht vorgesehen ist, in der sich die Flüssigkeit parallel zu den Schichtoberflächen ausbreitet und die mit der Flüssigkeitstransportstrecke in Fluidkontakt steht, parallel zu der Flüssigkeitstransportschicht die Signalbildungsschicht verläuft, die Signalbildungsschicht und die Flüssigkeitstransportschicht über eine Flüssigkeitsaustauschfläche miteinander in Fluidkontakt senkrecht zur Ebene der Basisschicht stehen und die Flüssigkeitstransportgeschwindigkeit der Flüssigkeitstransportschicht und der zeitliche Beginn der Signalbildungsreaktion in der Signalbildungsschicht derartig aufeinander abgestimmt sind, daß sich zunächst die Flüssigkeit in der Flüssigkeitstransportschicht ausbreitet und die Signalbildungsreaktion im wesentlichen danach stattfindet.

Der Begriff "Fluidkontakt" (engl. fluid contact) ist eine in der Testträgertechnik üblich gewordene Bezeichnung dafür, daß zwei Testschichten so zueinander angeordnet sind, daß zwischen ihnen ein Flüssigkeitsaustausch, meist aufgrund der Saugwirkung der Testschichten, möglich ist. Die Testschichten können dabei in unmittelbarem Kontakt zueinander stehen, oder der Fluidkontakt kann indirekt z.B. über dazwischenliegende saugfähige Schichten bewirkt werden.

Durch die erfindungsgemäße zeitliche Abstimmung wird bei einem Testträger mit Längstransport erreicht, daß eine homogen zusammengesetzte Flüssigkeitsfront aus der Transportschicht in die Signalbildungsschicht zu einem definierten Zeitpunkt eindringt. Dadurch wird eine homogene und zeitlich definierte Farbbildung erreicht. Es können extrem dünne Signalbildungsschichten mit einer hohen Konzentration von Signalbildungsreagenz verwendet werden, ohne daß dies zu Inhomogenitäten der Farbbildung führt.

Die Signalbildungsschicht und die Flüssigkeitstransportschicht können fest miteinander verbunden sein, soweit die Befestigungsart den Fluidkontakt im Bereich der Flüssigkeitsaustauschfläche nicht behindert. Vorzugsweise liegen beide Schichten im Bereich der Flüssigkeitsaustauschfläche lose aufeinander auf, und werden durch geeignete Maßnahmen, beispielsweise eine Niederhalterschicht aus Kunststoff, zusammengedrückt.

Die Flüssigkeitstransportschicht besteht aus einem saugfähigen Material, beispielsweise einem Papier, einer textilen Struktur (Gewebe, Vlies) oder, besonders bevorzugt, einer Kunststoffmembran. Jedes derartige Material hat Spalten oder Poren, in denen die Flüssigkeit durch Kapillarkräfte transportiert wird. Die Flüssigkeitstransportgeschwindigkeit wird bekanntermaßen durch die in der Schicht herrschenden Kapillarkräfte und durch den Strömungswiderstand der Schicht beeinflußt. Nähere Einzelheiten hierzu sind dem Fachmann bekannt bzw. aus entsprechenden Publikationen, beispielsweise der DE-A 36 43 516 zu entnehmen. Aufgrund der praktischen Bedingungen bei der Gestaltung von Testträgern ist es in sehr vielen Fällen jedoch nicht möglich, eine Flüssigkeitstransportschicht zu wählen, die sich sehr schnell füllt. In der Praxis werden vielmehr häufig Materialien eingesetzt in denen die Flüssigkeit verhältnismäßig langsam in Längsrichtung der Schicht transportiert wird. Entsprechend muß dann die Signalbildungsreaktion verzögert werden, um die Bedingung, daß sie im wesentlichen nach Füllung der Flüssigkeitstransportschicht beginnt, einzuhalten.

Dies kann beispielsweise dadurch geschehen, daß das Signalbildungs-Reagenzsystem ein zusätzliches Reagenz enthält, das eine Vorreaktion bewirkt, die nicht zu einer Signalbildung führt. In der Praxis wird bei analytischen Reaktionen der klinischen Chemie sehr häufig die enzymatisch katalysierte Umwandlung eines farbbildenden Substrats eines an der Reaktion beteiligten Enzyms als Signalbildungsreaktion eingesetzt. In diesem Falle kann eine Verzögerung der Farbbildung dadurch erreicht werden, daß zusätzlich zu dem farbbildenden Substrat ein nicht farbbildendes Substrat eingesetzt wird, das eine höhere Affinität zu dem Enzym hat, so daß zunächst überwiegend das nicht farbbildende Substrat reagiert und der Farbumschlag erst einsetzt, wenn das farbbildende Substrat weitgehend verbraucht ist. Diese Maßnahme wird in der DE-A 36 40 318 für einen anderen Anwendungszweck näher erläutert.

Besonders bevorzugt wird die Signalbildung jedoch dadurch verzögert, daß das Signalbildungsreagenz in der Signalbildungsschicht verzögert löslich ist. Das Signalbildungsreagenz selbst kann langsam löslich sein. Vorzugsweise wird das verzögerte Lösungsverhalten jedoch dadurch bewirkt, daß das Signalbildungsreagenz in eine verzögert lösliche Filmschicht eingebettet ist, die sich auf einem Träger in Form einer Folie oder eines Gewebes befindet. Gemäß einer weiteren besonders bevorzugten Ausführungsform ist dabei das Signalbildungsreagenz schneller löslich als die Filmschicht, in die es eingebettet ist. Dadurch wird erreicht, daß das Signalbildungsreagenz, wenn es aus der Signalbildungsschicht freigesetzt ist, in flüssiger Phase schnell und homogen reagiert. Es ist vorteilhaft, wenn dabei das Signalbildungsreagenz zumindest teilweise in die Flüssigkeitstransportschicht eindringt, so daß die Signalbildungsreaktion zum Teil oder -was besonders bevorzugt ist- sogar überwiegend in der Flüssigkeitstransportschicht abläuft.

Es ist ersichtlich, daß bei jedem der beschriebenen Verfahren zur Verzögerung der Signalbildungsreaktion diese in geringem Umfang schon einsetzt, während sich die Flüssigkeit noch in der Flüssigkeitstransportschicht ausbreitet. Es genügt, wenn die Farbbildungsreaktion in dem Sinne "im wesentlichen" nach der Flüssigkeitsausbreitung stattfindet, daß eine Anreicherung von Farbstoffen in der Flüssigkeitsfront, die in der Flüssigkeitstransportschicht in Längsrichtung des Testträgers strömt, vermieden und dadurch eine auf der ganzen Signalbildungsschicht homogene Signalbildung erreicht wird.

Um das gewünschte verzögerte Lösungsverhalten zu bewirken, enthält die Signalbildungsschicht bevorzugt quellbare Makromoleküle oder einen hydrophoben Filmbildner.

Quellbare Makromoleküle in diesem Sinne werden auch als Hydrokolloide bezeichnet. Es sind makromolekulare hydrophile Substanzen, die in Wasser löslich oder zumindest dispergier- und quellbar sind. Hierzu gehören insbesondere Polysaccharide wie z.B. Exudate (z.B. Gummi arabicum), Samenmehle (z.B. Johannisbrotkernmehl, Stärke), Extrakte aus Pflanzen (z.B. Pektine, Alginate), mikrobielle Polysaccharide (z.B. Xantangummi) und chemisch modifizierte Polysaccharide(z.B. Cellulose-Derivate).

Auch bestimmte Proteine sind zu dieser Substanzgruppe zuzurechnen, wie z.B. Skleroproteine und ihre Hydrolysate (z.B. Collagen, Crotein C), schwer lösliche Reserveproteine (z.B. Zein, gefälltes Casein), und hydrophob auftrocknende Proteine in entsprechend hohen Konzentrationen (z.B. Serumalbumin).

Als hydrophobe Filmbildner im Sinne der Erfindung werden wasserlösliche Polymere verstanden, die bei der Trocknung aus einer wässrigen Lösung einen verzögert anlösbaren Film bilden, in dem durch die Klebewirkung des Polymeren die Filminhaltsstoffe verfestigt und gebunden werden.

Die Auflösungseigenschaften solcher Filme können in weitem Maß durch Auswahl der Polymeren gesteuert werden. Hierzu gehören beispielsweise Polyvinylalkohole, wie sie unter dem Markenzeichen "Mowiol" von der Hoechst AG, Frankfurt, Bundesrepublik Deutschland (BRD), vertrieben werden, Polyäthylenoxyd, wie es unter dem Markennamen "Polyox" von der Union Carbide Corp., New York, USA, vertrieben wird, oder Acrylharze, wie sie von der Firma Roehm, Darmstadt, BRD unter dem Markenzeichen "Eudragid" erhältlich sind.

Die Erfindung eignet sich vor allem für einen Testträger zur Durchführung immunologischer Bestimmungen, wie im folgenden anhand eines in den Figuren schematisch dargestellten Ausführungsbeispiels näher erläutert wird. Es zeigen:

Fig. 1     eine perspektivische Darstellung eines erfindungsgemäßen Testträgers,

Fig. 2     feine Detailansicht aus Fig. 1

Der in den Figuren dargestellte Testträger 1 hat eine Basisschicht 2, auf der die übrigen Testschichten befestigt sind. In seiner Längsrichtung läßt sich der Testträger in einen Probenaufgabe- und Vorreaktionsbereich 4 und in einen Auswertebereich 6 unterteilen. In dem Probenaufgabe- und Vorreaktionsbereich 4 sind nebeneinander eine Konjugatschicht 8 und eine Flüssigkeitstransportschicht 9 auf der Basisschicht 2 mit Hilfe von Schmelzkleber 10 befestigt. Die Schicht 8 überlappt geringfügig die anschließende Schicht 9 um einen möglichst guten Fluidkontakt zwischen ihnen zu gewährleisten. Die Schichten 8 und 9 bestehen aus saugfähigem Material und bilden eine Flüssigkeitstransportstrecke, die sich von dem Probenaufgabe- und Vorreaktionsbereich 4 in den Auswertebereich 6 erstreckt.

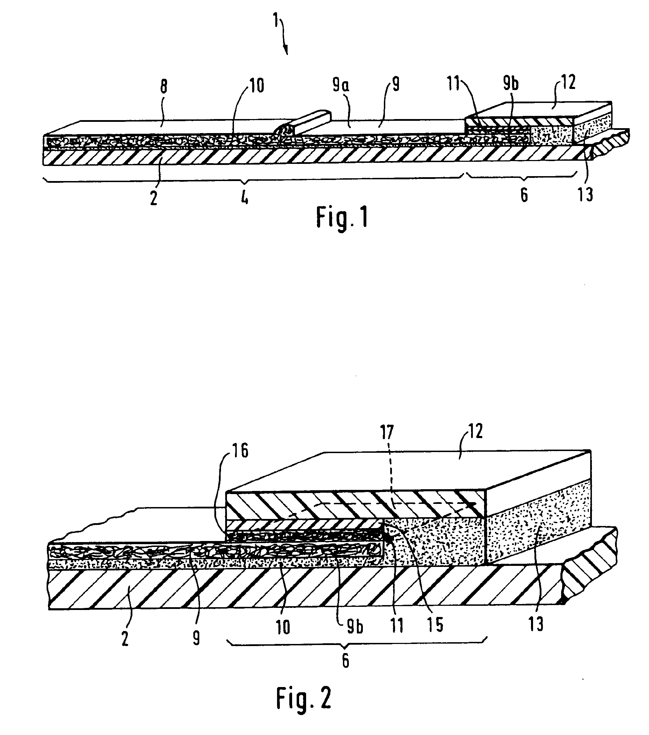

Im dargestellten Beispielsfall wird die Probe auf die Konjugatschicht 8 aufgegeben, wobei diese Schicht gleichzeitig dazu dient, eine erste Reaktionsstufe durchzuführen. Der Bereich 4 stellt deswegen einen kombinierten Probenaufgabe- und Vorreaktionsbereich dar. Alternativ könnte auch in Fig. 1 links von der Konjugatschicht 8 eine spezielle Probenaufgabeschicht vorgesehen sein, die einen von dem Vorreaktionsbereich separaten Probenaufgabebereich definiert.

In dem Auswertebereich 6 sind auf der Basisschicht 2, wie in Fig. 2 deutlicher zu erkennen ist, übereinander die Flüssigkeitstransportschicht 9, eine Signalbildungsschicht 11 und eine Niederhalterschicht 12 zu erkennen. Die Niederhalterschicht 12 besteht aus einer verhältnismäßig steifen Kunststoffolie. Sie ist mit Hilfe eines Schmelzkleberstreifens 13 entsprechend großer Schichtdicke an der Basisschicht 2 so befestigt, daß sie parallel zu ihr mit einem Abstand verläuft, der etwa der Gesamtdicke von Signalbildungsschicht 11 und Flüssigkeitstransportschicht 9 entspricht. Die Niederhalterschicht 12 hat eine ausreichende Steifigkeit, um die zwischen ihr und der Basisschicht 2 befindlichen Schichten so zusammenzudrücken, daß ein guter Fluidkontakt zwischen ihnen gewährleistet ist.

Zweckmäßigerweise sind neben der Farbbildungsschicht auf ihrer in Längsrichtung der Basisschicht 2 von dem Probenaufgabebereich abgewandten Seite (also in Fig. 1 rechts von der Signalbildungsschicht 11) keine weiteren saugfähigen Schichten vorgesehen. Die Signalbildungsschicht 11 steht also mit dem in Flüssigkeitstransportrichtung letzten Teilstück der Flüssigkeitstransportschicht 9 in Fluidkontakt.

Die Signalbildungsschicht 11 besteht in der dargestellten bevorzugten Ausführungsform aus einer Tragfolie 15,und einer darauf befindlichen verzögert löslichen Filmschicht 16, die ein Signalbildungsreagenz enthält.

Der in den Figuren dargestellte Testträger ist, wie erwähnt, insbesondere für immunologische Bestimmungen geeignet. Derartige Bestimmungen benutzen hochspezifische Bindungsreaktionen zwischen verschiedenen Spezies, die man als Bindungspartner bezeichnen kann. Immunologische Bindungspartner sind insbesondere Antikörper einerseits, sowie Antigene oder Haptene andererseits.

Für den Fall, daß ein in der Probe enthaltenes Antigen AG als Analyt bestimmt werden soll, ist beispielsweise folgender Testablauf typisch.

Die Probe wird auf die Konjugatschicht 8 aufgegeben. Die Konjugatschicht enthält ein lösliches Konjugat AKE eines mit dem AG spezifisch bindungsfähigen Antikörpers AK mit einem Enzym E. Durch die spezifische Bindungsreaktion entstehen Komplexe AG-AKE.

Überschüssiges AKE gelangt zusammen mit den AG-AKE-Komplexen in die Flüssigkeitstransportschicht 9. Diese enthält ein Antigen AGF in trägerfixierter Form. Das AGF ist mit dem Probenantigen identisch oder analog zu diesem, d.h. mit dem Antikörper des in der Konjugatschicht 8 enthaltenden AKE spezifisch bindungsfähig.

Das überschüssige freie Konjugat wird nun aufgrund der spezifischen Bindungsreaktion mit dem in der Schicht 9 fixierten Antigen trägerfixiert. Für die Funktion ist es wichtig, daß die Belegungsdichte des fixierten Antigens auf der Schicht 9 und zwar speziell in dem Teil 9a dieser Schicht, der sich in der Vorreaktionszone 5 befindet, hoch genug ist, daß praktisch das gesamte überschüssige Konjugat daran gebunden wird. Der Teil 9a der Schicht 9 wird deshalb als "Fixierungsschicht" bezeichnet. Nur die freien AG-AKE-Komplexe gelangen in den Teil 9b der Schicht 9, der sich in der Auswertezone 6 befindet. Dadurch entspricht die Menge der in der Auswertezone 6 eintreffenden AG-AKE-Komplexe (und damit die Menge des Markierungsenzyms) der Menge des Analyten.

Aufgrund der vorliegenden Erfindung wird der Teil 9b der Flüssigkeitstransportschicht 9 vollständig gefüllt, im wesentlichen bevor die Signalbildungsreaktion mit dem Signalbildungsreagenz in der Schicht 11 beginnt. Erst wenn sich die Flüssigkeit in der Schicht 9b vollständig ausgebreitet hat, beginnt die Signalbildungsreaktion, wobei dies,wie oben dargelegt, insbesondere dadurch erreicht wird, daß sich die Signalbildungsschicht 11 verzögert löst.

Erfindungsgemäß breitet sich die Flüssigkeit in der Schicht 9b zunächst "lateral", also in Richtung parallel zu den Oberflächen der Schicht (und damit in Längsrichtung des Testträgers) aus. Die Farbbildung in der Schicht 11 setzt im wesentlichen erst danach ein. Die Flüssigkeit dringt aus dem Teil 9b der Schicht 9 senkrecht zu deren Oberfläche durch die in Fig. 2 gestrichelt dargestellte Flüssigkeitsaustauschfläche 17 gleichmäßig in die Signalbildungsschicht ein, löst (im beschriebenen Beispielsfall) ein dort enthaltenes farbbildendes Substrat für das Markierungsenzym des Konjugats und katalysiert dessen farbbildende Reaktion. Der Farbumschlag ist damit ein Maß für die Konzentration des Enzyms und damit für die Konzentration des Analyten.

Die Fixierungsschicht 9a ist im dargestellten Fall ein Teil der Flüssigkeitstransportschicht 9. Es könnte auch eine getrennte Schicht als Fixierungsschicht verwendet werden, die mit der Flüssigkeitstransportschicht in Fluidkontakt steht. Die Verwendung einer einzigen Schicht, die sich teilweise als Fixierungsschicht in den Vorreaktionsbereich erstreckt und teilweise im Nachweisbereich den Transport parallel zu der Signalbildungsschicht bewirkt, ist jedoch besonders bevorzugt, weil sie einen einfachen Aufbau erlaubt und den Bedarf an Probenflüssigkeit reduziert. In diesem Fall muß das Schichtmaterial so gewählt sein, daß es eine hohe Belegungsdichte mit dem fixierten Antigen erlaubt und die Transportgeschwindigkeit relativ langsam ist, um die Fixierung des überschüssigen Konjugats zu ermöglichen. Hier ist es besonders wichtig, daß das Signalbildungsreagenz in der Signalbildungsschicht verzögert löslich ist.

Die Funktionsweise des Testträgers wurde beispielhaft für den Fall beschrieben, daß ein Antigen bestimmt werden soll. Ein analoger Testverlauf ist auch zur Bestimmung eines Antikörpers möglich, wobei dann ein Antigenkonjugat in der Schicht 8 und ein trägerfixierter analoger Antikörper in der Schicht 9 eingesetzt werden müßte.

Allgemein ist der erfindungsgemäße Testträger besonders für solche Bestimmungen geeignet, bei denen die Reaktionsfolge eine spezifische Bindungsreaktion zwischen einem mit der Konzentration des zu bestimmenden Bestandteils korrelierten ersten Bindungspartner (im Beispielsfall AG) und einem markierten zweiten Bindungspartner (im Beispielsfall AKE) unter Bildung beweglicher Komplexe (im Beispielsfall AG-AKE) und eine weitere spezifische Bindungsreaktion zwischen dem zweiten Bindungspartner (im Beispielsfall wiederum AKE) und einem zum ersten Bindungspartner analogen trägerfixierten dritten Bindungspartner (im Beispielsfall AGF) umfaßt. Dabei ist notwendig, daß der zweite und der dritte Bindungspartner,jeweils bezogen auf die Strömungsrichtung der Flüssigkeit,in dem Testträger vor dessen Nachweisbereich, also in einem außerhalb des Nachweisbereichs liegenden Vorreaktionsbereich angeordnet sind. Dadurch ist die spezifische Bindungsreaktion abgeschlossen, bevor die für die Analyse spezifischen freien Komplexe in den Nachweisbereich gelangen.

Der beschriebene immunologische Testablauf ist -abgesehen von den Besonderheiten der jeweiligen Erfindung- ähnlich dem in der DE-A 36 38 654 beschrieben. Auf diese Publikation wird daher ergänzend Bezug genommen.

Der erfindungsgemäße Testträger ist als Nachweiseinheit für einen Testkit zur Bestimmung eines Analyten im Stuhl besonders geeignet, wie er in EP-A-291843 beschrieben wird. Dieser Testkit hat eine Probensammeleinheit, in der aus den Stuhlproben durch Eluation mit Hilfe eines Eluationsmittels eine Flüssigkeit gewonnen wird, die den Analyten enthält. Die so gewonnene Probenflüssigkeit kann vorteilhaft mit dem Testträger gemäß der vorliegenden Erfindung untersucht werden.

Das folgende Beispiel 1 betrifft einen solchen Testträger, bei dem als Analyt human Serum Albumin (hSA) bestimmt wird, das aus einer Stuhlprobe eluiert wurde und ein Indikator für die Anwesenheit von Blut im Stuhl ist.

Beispiel 1:

Ein Testträger gemäß den Figuren 1 und 2 wird wie folgt hergestellt:

a) Konjugatschicht 8:

IgG <human Serum Albumin> wird mit β-Galaktosidase kovalent konjugiert. Dieses Konjugat wird in ein Glasfaservlies eingetränkt und getrocknet. Die Testschichtgröße auf dem Testträger beträgt 20 x 6 mm.

b) Flüssigkeitstransportschicht 9 (zugleich Fixierungsschicht):

An eine Membran aus hydrophilem Polyvinylidendifluorid (PVDF) der Firma Millipore (Bedford, USA), die unter dem Markennamen Immobilon AV vertrieben wird, wird hSA kovalent fixiert. Die Flächenkonzentration wird über die Konzentration in dem für den Tränkungsvorgang verwendeten Puffer auf 20 $\mu$g hSA/cm$^2$ eingestellt. Die Schichtgröße beträgt 20 x 6 mm.

c) Signalbildungsschicht 11:

Eine filmbildende Beschichtungsmasse wird hergestellt auf Basis von 0,6 % Ketrol F der Fa. Kelco, Hamburg, Bundesrepublik Deutschland (BRD) unter Zusatz von 2,5 % Methylcellulose 15 der Fa. Serva, Heidelberg, BRD. Sie enthält 12 mM ChlorphenolRot-β-Galaktosid (CPRG) und ist in HEPES gepuffert. Die Beschichtungsmasse wird in einer Filmschichtstärke von 200 $\mu$m auf eine 100 $\mu$m starke Tragfolie aus Pokalon der Fa. Lonza, Weil/Rh., BRD beschichtet. Die Schichtgröße beträgt 6 x 6 mm.

d) Niederhalterschicht 12:

Diese besteht aus einer 140 $\mu$m starken Pokalonfolie.

Als Basisschicht wird eine Polyesterfolie "Melinex" der Firma ICI, Frankfurt, BRD verwendet. Die Verklebung der Komponenten erfolgt mit Schmelzkleber Dynapol S 1358 der Fa. Dynamid Nobel, Troisdorf, BRD.

Die Flüssigkeitsfront bewegt sich in der Schicht 9 verhältnismäßig langsam, so daß die Ausbreitung in der Schicht nahezu 3 min. benötigt. Durch das verzögerte Lösungsverhalten der Substratschicht 11 wird dennoch eine völlig homogene Farbbildung in Abhängigkeit von der hSA-Konzentration erreicht, die eine Bestimmung dieser Konzentration mit guter Genauigkeit erlaubt.

In den Beispielen 2-4 werden verschiedene Rezepturen für eine Signalbildungsschicht 11 bzw. deren Filmschicht 16 gegenübergestellt und ihr Lösungsverhalten verglichen.

Beispiel 2:

Einer Basisrezeptur für einen Farbbildungsfilm (Substratfilm) bestehend aus

| 30 g | Keltrol F, ein Xanthan der Fa. Kelco, Brüssel, Belgien (1%ig in 50 mM HEPES, pH 7,0) |
| 17 g | CPRG (30 mM in $H_2O$) |
| 3 g | 200 mg Tween 20 (Fa. Serva, Heidelberg, BRD) + 50 mg $MgCl_2$ in 2,75 g $H_2O$ |

werden unterschiedliche Substanzen zugesetzt, um deren Verhalten bezüglich einer Verzögerung der Löslichkeit der an sich gut löslichen Xanthan-Filmschicht (DE-A 36 30 999) zu vergleichen. Es wird die Zeit verglichen, innerhalb der ein solcher Film nach Benetzung mit Wasser sich so weit gelöst hat, daß er vollständig abwischbar ist. Dabei ergaben sich folgende Resultate:

```
           Lösungsverzöger              abwischbar nach

a)              Basisrezeptur              sofort
b)    2 %      Methylcellulose 15
               (wasserlöslicher Cellulose-
               ether MG 14000, SERVA,Hei-
               delberg, BRD)               5 sec
c)    2 %      Ficoll 70
               (Kopolymer aus Sucrose und
               Epichlorhydrin, SERVA)      2 sec
d)    2 %      Crotein C
               (Collagenhydrolysat, Fa.
               Croda, Cheshire        )    2 sec
e)    2 %      Mowiol 18/88
               (teilweise verseifter Poly-
               vinylalkohol der Fa. Hoechst,
               Frankfurt, BRD)             2 sec
```

Die Verzögerung der Löslichkeit erscheint kurz im Verhältnis zu der in Beispiel 1 erwähnten langsamen Ausbreitung der Flüssigkeit in der Flüssigkeitstransportschicht 9. Dabei ist jedoch zu bedenken, daß das Abwischen eine mechanische Belastung der Schicht ist, die in dem Testträger nicht auftritt. Die hier erreichte Lösungsverzögerung ist deshalb in der Praxis ausreichend. Der Abwisch-Test erlaubt die Auswahl geeigneter Filmrezepturen.

Beispiel 3: Einfluß unterschiedlicher Mengen an Methylcellulose 15.

Der Basisrezeptur von Beispiel 1 werden unterschiedliche Mengen an Methylcellulose 15 zugegeben und die Abwischzeiten beobachtet. Es ergaben sich folgende Ergebnisse:

```
   Konzentration Methylcellulose 15      abwischbar nach
(0)        0                                 sofort
(1)        0,1 %                             sofort
(2)        0,5 %                             1 sec
(3)        1,0 %                             2 sec
(4)        2,0 %                             5 sec
(5)        5,0 %                            15 sec
```

Beispiel 4:

Ein anderer Film für eine Signalbildungsschicht 9 mit einer Anlösedauer von 10 sec läßt sich mit folgender Rezeptur unter Verwendung eines hydrophob antrocknenden Filmbildners herstellen:

```
30,0 g Mowiol  8/88    (23%ig in 20 mM HEPES,pH 7,2)
16,7 g CPRG-Lösung     (30 mM in 20 mM HEPES,pH 7,2)
 0,2 g Tween 20
 0,1 g MgCl₂
 3,0 g H₂O
```

**Patentansprüche**

1. Testträger zur analytischen Bestimmung eines Bestandteils einer flüssigen Probe, insbesondere für die Diagnose von Krankheiten, mittels einer auf dem Testträger ablaufenden Reaktionsfolge, mit mehreren Testschichten, umfassend eine Basisschicht (2), eine Flüssigkeitstransportstrecke (8,9) die sich von einem Probenaufgabebereich (4) in einen Auswertebereich (6) erstreckt, und eine Signalbildungsschicht (11), in der aufgrund einer Signalbildungsreaktion mit Hilfe eines Signalbildungs-Reagenzsystems eine für den zu bestimmenden Bestandteil charakteristische optisch nachweisbare Veränderung stattfindet, wobei sich mindestens ein Signalbildungsreagenz in der Signalbildungsschicht (11) befindet, **dadurch gekennzeichnet,** daß in dem Auswertebereich eine aus einem saugfähigen Material bestehende Flüssigkeitstransportschicht (9b) vorgesehen ist, die mit der Flüssigkeitstransportstrecke (8,9) in Fluidkontakt steht,
parallel zu der Flüssigkeitstransportschicht (9b) die Signalbildungsschicht (11) verläuft,
die Signalbildungsschicht (11) über eine Flüssigkeitsaustauschfläche (17) mit der Flüssigkeitstransportschicht (9b) in Fluidkontakt senkrecht zur Ebene der Basisschicht (2) steht und
die Flüssigkeitstransportgeschwindigkeit der Flüssigkeitstransportschicht (9b)und der zeitliche Beginn der Signalbildungsreaktion in der Signalbildungsschicht (11) derartig aufeinander abgestimmt sind, daß sich zunächst die Flüssigkeit in der Flüssigkeitstransportschicht (9b)ausbreitet und die Signalbildungsreaktion im wesentlichen danach stattfindet.

2. Testträger nach Anspruch 1, **dadurch gekennzeichnet,** daß die Signalbildungsschicht (11) und die Flüssigkeitstransportschicht (9b)im Bereich der Flüssigkeitsaustauschfläche (17) lose aufeinander aufliegen.

3. Testträger nach Anspruch 1, **dadurch gekennzeichnet,** daß das Signalbildungs-Reagenz in der Signalbildungsschicht(11) verzögert löslich ist.

4. Testträger nach Anspruch 3, **dadurch gekennzeichnet,** daß das Signalbildungsreagenz schneller löslich ist, als die Signalbildungsschicht (11), in die es eingebettet ist.

5. Testträger nach Anspruch 3, **dadurch gekennzeichnet,** daß die Signalbildungsschicht als Filmschicht (16) ausgebildet ist, in die das Signalbildungs-Reagenz eingebettet ist.

6. Testträger nach Anspruch 3, **dadurch gekennzeichnet,** daß die Signalbildungsschicht (11) quellbare makromolekulare Substanzen enthält.

7. Testträger nach Anspruch 3, **dadurch gekennzeichnet,** daß die Signalbildungsschicht (11) einen hydrophoben Filmbildner enthält.

8. Testträger nach Anspruch 1, **dadurch gekennzeichnet,** daß das Signalbildungs-Reagenz in der Signalbildungsschicht (11) ein farbbildendes Substrat für ein an der Analysereaktion beteiligtes Enzym ist.

9. Testträger nach Anspruch 8, **dadurch gekennzeichnet,** daß das Substrat Chlorphenolrot-β-galactosid ist.

10. Testträger nach Anspruch 1, **dadurch gekennzeichnet,** daß eine Niederhalterschicht (12) an der Basisschicht (2) befestigt ist und die Signalbildungsschicht (11) und die Flüssigkeitstransportschicht (9b) im Bereich der Flüssigkeitsaustauschfläche (17) zwischen der Basisschicht (2) und der Niederhalterschicht (12) so angeordnet sind, daß sie zusammengedrückt werden.

11. Testträger nach Anspruch 1, **dadurch gekennzeichnet,** daß die Reaktionsfolge eine spezifische Bindungsreaktion zwischen einem mit der Konzentration des zu bestimmenden Bestandteils korrelierten ersten Bindungspartner und einem markierten zweiten Bindungspartner unter Bildung beweglicher Komplexe,und eine spezifische Bindungsreaktion zwischen dem zweiten Bindungspartner und einem zu dem ersten Bindungspartner analogen trägerfixierten dritten Bindungspartner unter Bildung fixierter Komplexe umfaßt,
daß die Signalbildungsreaktion eine Reaktion zwischen dem Signalbildungs-Reagenz und den freien Komplexen einschließt und,
daß der zweite und der dritte Bindungspartner in einem außerhalb des Nachweisbereiches (6) angeordneten Vorreaktionsbereich so angeordnet sind, daß die spezifischen Bindungsreaktionen abgeschlossen sind, bevor die freien Komplexe in den Nachweisbereich gelangen.

12. Testträger nach Anspruch 11, **dadurch gekennzeichnet,** daß die Flüssigkeitstransportschicht (9b)sich aus dem Nachweisbereich (6) in den Vorreaktionsbereich erstreckt und ihr in dem Vorreaktionsbereich befindlicher Teil (9a) eine Fixierungsschicht bildet, an der der trägerfixierte dritte Bindungspartner fixiert ist.

## Claims

1. Test carrier for the analytical determination of a component of a liquid sample, especially for the diagnosis of diseases, by means of a reaction sequence taking place on the test carrier
the test carrier having a plurality of test layers comprising
a base layer (2), a liquid transport path (8, 9), which extends from a sample application region (4) to an evaluation region (6), and a signal formation layer (11) in which, on the basis of a signal formation reaction, with the help of a signal formation reagent system, an optically detectable change characteristic for the component to be determined takes place, at least one signal formation reagent being present in the signal formation layer (11),
characterized in that
in the evaluation region, there ist provided a liquid transport layer (9b) consisting of an absorbent material which is in fluid contact with the liquid transport path (8, 9),
the signal formation layer (11) runs parallel to the liquid transport layer (9b),
the signal formation layer (11) is in liquid contact with the liquid transport layer (9b) via a liquid exchange surface (17) perpendicular to the plane of the base layer (2) and
the speed of liquid transport of the liquid transport layer (9b) and the chronological commencement of the signal formation reaction in the signal formation layer (11) are adapted to one another in such a manner that the liquid first spreads out in the liquid transport layer (9) and the signal formation reaction takes place substantially thereafter.

2. Test carrier according to claim 1, characterized in that the signal formation layer (11) and the liquid transport layer (9b) lie loosely upon one another in the region of the liquid exchange surface (17).

3. Test carrier according to claim 1, characterized in that the signal formation reagent in the signal formation layer (11) is retardedly soluble.

4. Test carrier according to claim 3, characterized in that the signal formation reagent is more rapidly soluble than the signal formation layer (11) in which it is embedded.

5. Test carrier according to claim 3, characterized in that the signal formation layer is formed as a film layer (16) in which the signal formation reagent is embedded.

6. Test carrier according to claim 3, characterized in that the signal formation layer (11) contains a water-swellable macromolecular substance.

7. Test carrier according to claim 3, characterized in that the signal formation layer (11) contains a hydrophobic film forming material.

8. Test carrier according to claim 1, characterized in that the signal formation reagent in the signal formation layer (11) is a colour-forming substrate for an enzyme participating in the analysis reaction.

9. Test carrier according to claim 8, characterized in that the substrate is chlorophenol red-$\beta$-galactoside.

10. Test carrier according to claim 1, characterized in that a holding-down layer (12) is fixed on to the base layer (2) and the signal formation layer (11) and the liquid transport layer (9b) are so arranged in the region of the liquid exchange surface (17) between the base layer (2) and the holding-down layer (12) that they are pressed together.

11. Test carrier according to claim 1, characterized in that
the reaction sequence comprises a specific binding reaction between a first binding partner correlated with the concentration of the component to be determined and a labelled second binding partner resulting in the formation of mobile complexes, and a specific binding reaction between the second binding partner and a carrier-fixed third binding partner analogous to the first binding partner resulting in the formation of fixed complexes,
the signal formation reaction includes a reaction between the signal forming reagent and the free complexes and
the second and third binding partners are so arranged in a pre-reaction region which is located outside of the detection region (6) that the specific binding reactions are substantially completed before the free complexes pass into the detection region.

12. Test carrier according to claim 11, characterized in that the liquid transport layer (9b) extends from the detection region (6) into the pre-reaction region and the part (9a) thereof present in the pre-reaction region forms a fixing layer to which the carrier-fixed third binding partner is fixed.


**Revendications**

1. Bande de test pour la détermination analytique d'un constituant d'un échantillon liquide, en particulier pour le diagnostic de maladies, au moyen d'une suite de réactions se déroulant sur le support de test, celui-ci comportant plusieurs couches de test comprenant
une couche de base (2), une voie de transport de liquide (8,9) qui s'étend depuis une zone d'application de l'échantillon (4) jusque dans une zone d'évaluation (6), et une couche de formation de signal (11) dans laquelle se déroule, par suite d'une réaction de formation de signal à l'aide d'un système de réactifs de formation de signal, une variation optiquement détectable, caractéristique du constituant à déterminer, au moins l'un des réactifs de formation de signal se trouvant dans la couche de formation de signal (11),
caractérisé en ce que dans la zone d'évaluation, il est prévu une couche de transport de liquide (9b) constituée d'un matériau absorbant, qui est en contact de fluide avec la voie de transport de liquide (8,9), la couche de formation de signal (11) s'étend parallèlement à la couche de transport de liquide (9b), la couche de formation de signal (11) est en contact de fluide avec la couche de transport de liquide (9b) par l'intermédiaire d'une surface d'échange de liquide (17), perpendiculairement au plan de la couche de base (2), et la vitesse de transport du liquide dans la couche de transport de liquide (9b) et le début de la réaction de formation de signal dans la couche de formation de signal (11) sont ajustés réciproquement de façon que le liquide se répande d'abord dans la couche de transport de liquide (9b) et que la réaction de formation de signal se déroule essentiellement après.

2. Support de test selon la revendication 1, caractérisé en ce que la couche de formation de signal (11) et la couche de transport de liquide (9b) sont superposées librement dans la zone d'échange de liquide (17).

3. Support de test selon la revendication 1, caractérisé en ce que le réactif de formation de signal présente une solubilité retardée dans la couche de formation de signal (11).

4. Support de test selon la revendication 3, caractérisé en ce que le réactif de formation de signal se dissout plus rapidement que la couche de formation de signal (11) dans laquelle il est inclus.

5. Support de test selon la revendication 3, caractérisé en ce que la couche de formation de signal est conçue sous forme de couche de film (16) dans laquelle le réactif de formation de signal est inclus.

6. Support de test selon la revendication 3, caractérisé en ce que la couche de formation de signal (11) comprend des substances macromoléculaires susceptibles de gonfler.

7. Support de test selon la revendication 3, caractérisé en ce que la couche de formation de signal (11) comprend un filmogène hydrophobe.

8. Support de test selon la revendication 1, caractérisé en ce que le réactif de formation de signal dans la couche de formation de signal (11) est un substrat chromogène d'une enzyme participant à la réaction d'analyse.

9. Support de test selon la revendication 8, caractérisé en ce que le substrat est le rouge de chlorophénol-β-galactoside.

10. Support de test selon la revendication 1, caractérisé en ce qu'une couche de pression (12) est fixée sur la couche de base (2) et que la couche de formation de signal (11) et la couche de transport de liquide (9b) sont disposées, dans la zone de la surface d'échange de liquide (17), entre la couche de base (2) et la couche de pression (12), de façon à être pressées l'une contre l'autre.

11. Support de test selon la revendication 1, caractérisé en ce que la suite des réactions comprend une réaction de liaison spécifique entre un premier partenaire de liaison en corrélation avec la concentration du constituant à déterminer et un second partenaire de liaison marqué, avec formation de complexes mobiles, et une réaction de liaison spécifique entre le second partenaire de réaction et un troisième partenaire de réaction fixé sur un support, analogue au premier partenaire de réaction, avec formation de complexes fixes, et que la réaction de formation de signal comprend une réaction entre le réactif de formation de signal et les complexes libres, les second et troisième partenaires de liaison étant disposés dans une zone de réaction préliminaire extérieure à la zone de détection (6) de façon à exclure les réactions de liaison spécifiques, avant que les complexes libres ne parviennent dans la zone de détection.

12. Support de test selon la revendication 11, caractérisé en ce que la couche de transport de liquide (9b) s'étend depuis la zone de détection (6) jusque dans la zone de réaction préliminaire, et sa partie (9a), se trouvant dans la zone de réaction préliminaire, constitue une couche de fixation, sur laquelle est fixé le troisième partenaire de liaison fixé sur un support.

Fig. 1

Fig. 2